# EUROPEAN PATENT SPECIFICATION

(11) **EP 2 191 820 B1**
(45) Date of publication and mention of the grant of the patent: **28.09.2011**
(21) Application number: 09176632.9
(22) Date of filing: 20.11.2009
(51) Int. Cl.: A61K 9/08, A61K 31/54

(54) **Pharmaceutical formulations of meloxicam**
Pharmazeutische Formulierungen von Meloxicam
Formulations pharmaceutiques de méloxicam

(30) Priority: 01.12.2008 TR 200809200
(43) Date of publication of application: 02.06.2010
(73) Proprietor: Sanovel Ilac Sanayi ve Ticaret A.S., 34398 Istanbul (TR)
(72) Inventor: Turkyilmaz, Ali, 34398, Istanbul (TR); Turp, Hasan Ali, 34398, Istanbul (TR); Akdogan, Devdet, 34398, Istanbul (TR)
(74) Representative: Sevinç, Erkan

(56) References cited:
- WO-A-99/09988
- WO-A-2004/037264
- US-A1- 2002 035 107
- US-A1- 2007 092 539
- LUGER P ET AL: "STRUCTURE AND PHYSICOCHEMICAL PROPERTIES OF MELOXICAM, A NEW NSAID" EUROPEAN JOURNAL OF PHARMACEUTICAL SCIENCES, ELSEVIER, AMSTERDAM, NL, vol. 4, no. 3, 1 January 1996 (1996-01-01) , pages 175-187, XP001179121 ISSN: 0928-0987

## Description

### Technical Aspect

This invention is a novel pharmaceutical formulation of aqueous EDTA (Ethylene diamine tetraacetic acid) free solution of meloxicam in combination with meglumin for administration by oral or parenteral route, comprising one or more pharmaceutically acceptable excipients which is comprising N,N dimethylacetamide and propylene glycol for treating mammals, preferably animals.

More specifically, aqueous EDTA free solution of meloxicam is comprising N,N dimethylacetamide and propylene glycol in a raito of between 1:2 to 1:15 (w/w). Particularly preferred ratio is 1:5 (w/w).

### Background of the Invention

Meloxicam, an oxicam derivative, is a member of the enolic acid group of nonsteroidal anti-inflammatory drugs (NSAIDs). It is reported to be a selective inhibitor of cyclooxygenase-2 (COX-2) and exerts potent anti-inflammatory, anti-rheumatismal and antipyretic activity.

The chemical name of meloxicam is as 4-hydroxy-2-methyl-*N*-(5-methyl-2-thiazolyl)-2*H-*1,2-benzothiazine-3-carboxamide-1,1-dioxide and its chemical structure is shown in the Formula 1.

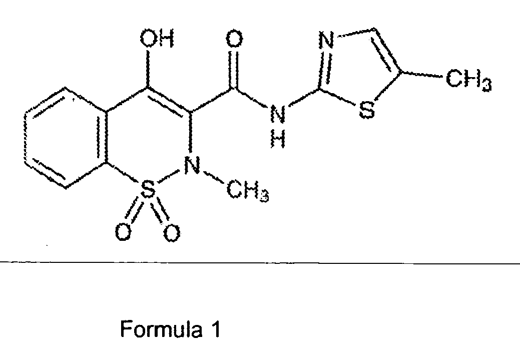

Meloxicam is a hydrophobic drug and diffucult to dissolve in aqueous solution. Meglumine can increase the solubility of meloxicam in aqueous solution (Zhao, J., Zhang, J.-S., Journal of China Pharmaceutical University, "Study on the solubilization of meloxicam in meglumine aqueous solution ", 2003, Vol.34, No.5, abstract). Meglumine is an organic base used as a pH-adjusting agent and solubilizing agent *(*Handbook of pharmaceutical excipients fourth edition, Rowe, Raymond C., Sheskey, Paul J., Weller, Paul J., pages 381 and 382*)*.

In prior art, there are many patents which referred meloxicam use with meglumin salts. US 4 233 299 (Boehringer Ingelheim GmbH) 16.12.1977, describes meloxicam and the sodium and meglumine salt (N-methyl-D-glucamine salt) thereof. Also this application shows, example of 0,2% injectable solution of meloxicam consisting of the meglumine salt of the active substance, sodium chloride and water.

EP application EP 0 945 134 A1 (Boehringer Ingelheim Pharma KG) 27.03.1998, discloses the pH-dependent solubility characteristics of meloxicam and its salts, i.e. the sodium salt, the ammonium salt and the meglumine salt, in aqueous solution.

EP 1 299 107 B1 (Boehringer Ingelheim Vetmedica GmbH) 20.06.2000, relates to an aqueous cyclodextrin-free solution of meloxicam which is suitable for oral or parenteral administration, containing a pharmacologically acceptable meloxicam salt of an organic or inorganic base and one or more suitable excipients selected from EDTA, citric acid, lecithin, gluconic acid, tartaric acid and phosphoric acid or the salts thereof. The formulation which contains a high concentration of active substance in a particle-free solution and is stable over the long term is suitable for treating pain, inflammation, fever, acute mastitis, diarrhoea, lameness, problems with the locomotor apparatus, and respiratory complaints in animals, preferably acute mastitis, diarrhoea, lameness, problems with the locomotor apparatus and respiratory complaints in large farm animals.

US application US 2003 0 119 825 A1 (Boehringer Ingelheim Vetmedica GmbH) 12.12.2001, describes a highly concentrated stable particle-free meloxicam solution suitable for administration by needleless injection containing from 35 to 100mg/ml of dissolved meloxicam salt and one or more suitable additives for treating respiratory diseases and inflammation in mammals.

PCT application WO 05/105101 A1 (Boehringer Ingelheim Vetmedica GmbH) 29.04.2004, relates to use of a formulation containing meloxicam or pharmacologically acceptable meloxicam salt of an organic or inorganic base, one or more vehicles and one or more suitable additives for preparing a veterinary medical composition for intramammary treatment of inflammatory diseases in mammals, particularly mastitis. PCT application WO 06/000306 A1 (Boehringer Ingelheim Vetmedica GmbH) 23.06.2004, relates to use of a formulation containing meloxicam or pharmacologically acceptable meloxicam salt of an organic or inorganic base, one or more vehicles and one or more suitable additives for preparing a veterinary medical composition for treatment of mild and/or moderate mastitis cases.

Meloxicam is practically insoluble in water and very slightly soluble in alcohol. Meglumine can increase the solubility of meloxicam in aqueous solution but it is not enough to derive an absolute and clear aqueous solution. In order to have this, heating process is needed or other solubilising agents are used. In prior art, a heating process at 90°C is used for preparing a pharmaceutical formulation of an aqueous solution of meloxicam.

EDTA is used to form stable water-soluble complexes. In this invention, we claim a novel and improved aqueous solution without using EDTA to form a stable pharmaceutical formulation and without using heating process at 90°C we obtain an appropriate solution of meloxicam in combination with meglumine.

One of the embodiments of this invention is to provide a manufacture process of stabilized aqueous EDTA free solution of meloxicam with meglumine without using the heating process for administration by oral or parenteral route, comprising one or more pharmaceutically acceptable excipients which is comprising N,N dimethylacetamide and propylene glycol.

This process is also environmental friendly because of its short duration and not using heating process in high degrees.

### Description of the invention

This invention is an aqueous EDTA free meloxicam solutions which comprise, meglumin and certain excipients, or excipient complex which is selected from the group comprising of N,N dimethylacetamide, propylene glycol, povidone, dimethyl ether, ethyl acetate, polyethylene glycol.

Surprisingly, it has been found that, when N,N dimethylacetamide and propylene glycol is used there is no need to use a heating step to make meloxicam dissolved easily. Also without using heating process we are more certain about the solution's long-term stability.

The problem underlying the present invention is to provide an aqueous EDTA free solution of meloxicam which is highly dissolved without using the heating process. Surprisingly, it has been found that, addition of N,N dimethylacetamide and propylene glycol to the present pharmaceutical formulation, an appropriate solution of meloxicam is obtained.

Therefore, several studies are done to reach the best solubility of meloxicam and meglumin complex in appropriate criterias. As a result of this, an appropriate solubility of the formulation by using the complex of N,N dimethylacetamide and propylene glycol is obtained preferably in a ratio of between 1:2 to 1:15 (w/w). Particularly preferred ratio is 1:5 (w/w).

In preferred embodiments, aqueous EDTA free solution of meloxicam of the present invention comprising;
1.0 to 8.0 % of meloxicam
1.0 to 5.0 % of meglumine
5.0 to 30.0 % of N,N dimethyl acetamide
50.0 to 90.0 % of propylene glycol
01. to 1.5 % of povidone K-17

It may be advantegous if the formulation containing an aqueous medium according to the invention has a pH value in the alkaline range. In the more alkaline region the meloxicam containing formulation tends to be a true aqueous solution whereas in the more acidic region it tends to be a suspension. The formulation of this invention should have a pH range from 8 to 10, preferably from 8 to 9.

Therefore, according to the prior art the buffer substances used to maintain a pH value of 8 to 10, preferably from 8 to 9. In this invention surprisingly we obtained the optimum pH without using a buffer substance. N,N dimethylacetamide is a basic substance and addition of meglumin and itself helped to obtain the optimum pH.

The pharmaceutical formulations of the invention include solutions, suspensions, any kind of injection formulations, e.g. such as intracutaneous or subcutaneous needleless injection or ready to use syringes, or injection formulations for parenteral application, such as i.v. or i.m. injection. The preparation of pharmaceutical forms of this kind is well-known per se from the prior art.

The solubilisers may also be used, for example propyleneglycol, polyethyleneglycols, polyoxyethylene-polyoxypropylene copolymers, propylene carbonate, polyoxyl 35 castor oil, castor oil, polysorbate, propyleneglycol monostearate, glycofurol, glycerol, sorbitol, mannitol, xylitol, povidone, N,N-dimethylacetamide and lecithin. Particularly preferred are propyleneglycol, N,N-dimethylacetamide, povidone, polyethyleneglycols but especially propyleneglycol and N,N-dimethylacetamide.

One embodiment of the invention comprises, in addition to the meloxicam in combination with meglumin, propyleneglycol, N,N-dimethylacetamide, polyvinylpyrrolidone but particularly propyleneglycol and N,N-dimethylacetamide as solubiliser.

The other embodiment of this invention is the preservatife use of propyleneglycol when it is used in the range of %15 to 30 *(*Handbook of Pharmaceutical Excipients 4th edition, Rowe, Raymond C., Sheskey, Paul J., Weller, Paul J., pages 521-523*)*.

It is known that, in common with other edetates, sodium edetate (disodium EDTA, trisodium EDTA, tetrasodium EDTA) may cause gastrointestinal effects. Pain at the site of injection and thrombophlebitis may also occur. Other adverse effects include fever, skin rashes, hypotension and hyperuricaemia, nephrotoxicity has also been reported, particularly following overdosage. Hypocalemia can occur, particularly if sodium edetate is infused too rapidly or in too concentrated a solution and tetany, convulsions, respiratory arrest and cardiac arrhythmias may result. (Sean C Sweetman, Martindale The Complete Drug Reference, thirty-fifth edition 2007, Vol. 1, page 1318*)* Hence it may need caution for using EDTA in highly concentrated injectable formulations.

The most serious of adverse effect of CaEDTA is renal toxicity (renal tubular necrosis), also can cause depression and GI symptoms (vomiting, diarrhea). Animals with symptoms of cerebral edema should not be overhydrated. *(*Veterinary Drug Handbook, 3rd Ed., Ames, lowa State University Press, Pages 289-290*)*

It is known from the prior art that application of EDTA in pharmaceutical formulations is to form stable water-soluble complexes. The main advantage of this present invention is not using EDTA to form a good stabilization

The solution may have a long-term shelf-life of 24 months or more at ambient temperature, its original packaging.

Without using EDTA as a stabiliser we achieved good stability results, details of the stability test results can be found in Table 1 which follow:

**Table 1**

| **Stability Test Results** | | | |
|---|---|---|---|
| **Test No.** | **Storage conditions (°C/% relative humidty)** | **Storage time (months)** | **Meloxicam content (mg/ml)** |
| **01K07** | 25°C±2°C | 0 | 19.7 |
| | 25°C±2°C / %60±%5 | 3 | 20.1 |
| | 25°C±2°C / %60±%5 | 6 | 20.1 |
| | 30°C±2°C / %65±%5 | 3 | 20.5 |
| | 30°C±2°C / %65±%5 | 6 | 20.5 |
| | 40°C±2°C / %75±%5 | 3 | 20.2 |
| | 40°C±2°C / %75±%5 | 6 | 20.1 |
| **02K07** | 25°C±2°C | 0 | 19.5 |
| | 25°C±2°C / %60±%5 | 3 | 20.0 |
| | 25°C±2°C / %60±%5 | 6 | 20.5 |
| | 30°C±2°C / %65±%5 | 3 | 20.5 |
| | 30°C±2°C / %65±%5 | 6 | 20.3 |
| | 40°C±2°C / %75±%5 | 3 | 20.5 |
| | 40°C±2°C / %75±%5 | 6 | 20.3 |

| | | | |
|---|---|---|---|
| **mg/ml limit; 20.0 mg/ml ± 2.0 (18.0 - 22.0 mg/ml)** | | | |

The formulation according to the invention overcomes the problem arising from the prior art of providing an injectable solution of the active substance meloxicam in combination with meglumine comprising N,N dimethylacetamide and propylene glycol, preferably in a ratio of between 1:2 to 1:15 (w/w) and particularly preferred ratio is 1:5 (w/w) which is also suitable for treating large farm animals, by permitting a high concentration of active substance in EDTA free aqueous solution which is stable over the long term, having the formulation described hereinafter.

This invention is further defined by reference to the following examples. Although the example is not intended to limit the scope of the present invention, it should be considered in the light of the description detailed above.

### Example 1 : meloxicam solution

| **Ingredients** | **Amount (mg/ml)** |
|---|---|
| Meloxicam | 20.0 |
| Meglumine | 12.5 |
| N,N dimethyl acetamide | 80.0 |
| Propylene glycol | 200.0 |
| Povidone K-17 | 2.0 |
| Water for injections | q.s.p. 1 ml |

### Example 2 : meloxicam solution

| **Ingredients** | **Amount (mg/ml)** |
|---|---|
| Meloxicam | 20.0 |
| Meglumine | 12.5 |
| N,N dimethyl acetamide | 80.0 |
| Propylene glycol | 400.0 |
| Povidone K-17 | 2.0 |
| Water for injections | q.s.p. 1 ml |

### Example 3 : meloxicam solution

| **Ingredients** | **Amount (mg/ml)** |
|---|---|
| Meloxicam | 20.0 |
| Meglumine | 12.5 |
| N,N dimethyl acetamide | 30.0 |
| Propylene glycol | 200.0 |
| Povidone K-17 | 2.0 |
| Water for injections | q.s.p. 1 ml |

One of the main embodiments of the present invention is its process for the preparation of the EDTA free aqueous solution which comprises the steps:
a. meglumin and povidon K 17 are added to an amount of water for injection,
b. propyleneglycol and N,N dimethylacetamid are added to this mixture and mixed until homogenization,
c. meloxicam is added to the solution and stirred until it dissolves and diluted to its volume with injectable water
d. the solution is filtered out a 0.2 µm filter under aseptic conditions and sterilized,

This process takes place under nitrogen gaseous conditions. The crucial point of the process is not including a heating step at 90 °C.

The formulation according to the invention should have a pH of between 8 and 10, preferably between 8 and 9 without using a buffer substance.

The formulation according to the invention is suitable for treating pain, inflammation, fever, acute mastitis, diarrhoea, lameness, problems with the locomotor apparatus and respiratory complaints in animals. The treatment may be given in conjunction with antibiotic therapy.

The formulation according to the invention is suitable for treating mammals, preferably animals, more particularly farm animals.

## Claims

1. Aqueous EDTA free solution of meloxicam in combination with meglumin for administration by oral or parenteral route, comprising one or more pharmaceutically acceptable excipients, **characterised in that** the solution is comprising N,N dimethylacetamide and propylene glycol.

2. Aqueous EDTA free solution according to the claim 1, **characterised in that** the solution of N,N dimethylacetamide and propylene glycol is in a ratio of 1:2 to 1:15 (w/w).

3. Aqueous EDTA free solution according to the claim 1 and 2, **characterised in that** the solution of N,N dimethylacetamide and propylene glycol is in a ratio of 1:5 (w/w).

4. Aqueous EDTA free solution of meloxicam according to claims 1 to 3, **comprising**;
a. 1.0 to 8.0 % of meloxicam
b. 1.0 to 5.0 % of meglumin
c. 5.0 to 30.0 % of N,N dimethly acetamide
d. 50.0 to 90.0 % of propylene glycol
e. 0.1 to 1.5 % of povidone
f. Injectable water

5. Aqueous EDTA free solution according to claims 1 to 4, **characterised in that** it has a pH of between 8.0 and 10 without using a buffer substance.

6. Aqueous EDTA free solution according to any preceding claims, **characterised in that** it has a long term shelf-life of (24 months) or more at ambient temperature its original packaging.

7. A process for the preparation of the aqueous EDTA free solution, **comprising** the steps:
a. Addition of meglumin and Povidon,
b. Addition of propylen glycol and N,N dimethylacetamid,
c. Addition of meloxicam,
d. Filtration

8. A process for the preparation of the aqueous EDTA free solution of claim 7, **characterised in that** it does not include a heating step.

9. Use of aqueous EDTA free solution according to any preceding claims for preparing a pharmaceutical compositon for treating pain, inflammation, fever and respiratory complaints in mammals, preferably animals.

## Patentansprüche

1. Wässrige EDTA-freie Lösung von Meloxicam in Kombination mit Meglumin zur Verabreichung über den oralen oder parenteralen Weg, die einen oder mehrere pharmazeutisch verträgliche Hilfsstoffe umfasst, **dadurch gekennzeichnet, dass** die Lösung N,N-Dimethylacetamid und Propylenglykol umfasst.

2. Wässrige EDTA-freie Lösung nach Anspruch 1, **dadurch gekennzeichnet, dass** die Lösung von N,N-Dimethylacetamid und Propylenglykol in einem Verhältnis von 1:2 zu 1:15 (w/w) vorliegt.

3. Wässrige EDTA-freie Lösung nach Anspruch 1 und 2, **dadurch gekennzeichnet, dass** die Lösung von N,N-Dimethylacetamid und Propylenglykol in einem Verhältnis von 1:5 (w/w) vorliegt.

4. Wässrige EDTA-freie Lösung von Meloxicam nach den Ansprüchen 1 bis 3, umfassend:
a. 1,0 bis 8,0% Meloxicam
b. 1,0 bis 5,0% Meglumin
c. 5,0 bis 30,0% N,N-Dimethylacetamid
d. 50,0 bis 90,0% Propylenglykol
e. 0,1 bis 1,5% Povidon
f. Injizierbares Wasser

5. Wässrige EDTA-freie Lösung nach den Ansprüchen 1 bis 4, **dadurch gekennzeichnet, dass** sie einen pH von zwischen 8,0 und 10 ohne Verwendung einer Puffersubstanz aufweist.

6. Wässrige EDTA-freie Lösung nach einem der vorangehenden Ansprüche, **dadurch gekennzeichnet, dass** sie eine Langzeithaltbarkeitsdauer ihrer ursprünglichen Verpackung von 24 Monaten oder mehr bei Umgebungstemperatur aufweist.

7. Verfahren zur Herstellung der wässrigen EDTA-freien Lösung, umfassend die Schritte:
a. Zugabe von Meglumin und Povidon,
b. Zugabe von Propylenglykol und N,N-Dimethylacetamid,
c. Zugabe von Meloxicam,
d. Filtration

8. Verfahren zur Herstellung der wässrigen EDTA-freien Lösung nach Anspruch 7, **dadurch gekennzeichnet, dass** sie keinen Erhitzungsschritt einschließt.

9. Verwendung einer wässrigen EDTA-freien Lösung nach einem der vorangehenden Ansprüche zur Herstellung einer pharmazeutischen Zusammensetzung zur Behandlung von Schmerz, Entzündung, Fieber und Atembeschwerden bei Säugern, vorzugsweise Tieren.

## Revendications

1. Solution aqueuse de méloxicam, exempte d'EDTA, en combinaison avec du méglumine, pour l'administration par voie orale ou parentérale, comprenant un ou plusieurs excipients pharmaceutiquement acceptables, **caractérisée en ce que** la solution comprend de la N,N-diméthylacétamide et du propylène glycol.

2. Solution aqueuse, exempte d'EDTA, selon la revendication 1, **caractérisée en ce que** la solution de N,N-diméthylacétamide et de propylène glycol est en des proportions de 1:2 à 1:15 (poids/poids).

3. Solution aqueuse, exempte d'EDTA, selon la revendication 1 et 2, **caractérisée en ce que** la solution de de N,N-diméthylacétamide et de propylène glycol est en une proportion de 1:5 (poids/poids).

4. Solution aqueuse de méloxicam, exempte d'EDTA, selon les revendications 1 à 3, comprenant :
a. 1,0 à 8,0% de méloxicam
b. 1,0 à 5,0% de méglumine
c. 5,0 à 30,0 % de N,N-diméthylacétamide
d. 50,0 à 90,0 % de propylène glycol
e. 0,1 à 1,5% de povidon
f. de l'eau injectable

5. Solution aqueuse, exempte d'EDTA, selon les revendications 1 à 4, **caractérisée en ce qu'**elle a un pH entre 8,0 et 10 sans utilisation d'une substance tampon.

6. Solution aqueuse, exempte d'EDTA, selon l'une quelconque des revendications précédentes, **caractérisée en ce qu'**elle a une durée de vie à long terme de 24 mois ou davantage à température ambiante dans son emballage d'origine.

7. Un procédé pour la préparation d'une solution aqueuse, exempte d'EDTA, comprenant les étapes de :
a. Ajout de méglumine et de Povidon,
b. Ajout de propylène glycol et de N,N-diméthylacétamide
c. Ajout de méloxicam
d. Filtration

8. Un procédé pour la préparation d'une solution aqueuse, exempte d'EDTA, selon la revendication 7, **caractérisée en ce qu'**elle ne comprend pas d'étape de chauffage.

9. Utilisation d'une solution aqueuse, exempte d'EDTA, selon l'une quelconque des revendications précédentes, pour la préparation d'une composition pharmaceutique pour le traitement de la douleur, de l'inflammation, de la fièvre et des troubles respiratoires des mammifères, de préférence des animaux.
